# EUROPEAN PATENT SPECIFICATION

(11) **EP 2 623 068 B1**
(45) Date of publication and mention of the grant of the patent: **23.03.2022**
(21) Application number: 10857899.8
(22) Date of filing: 29.09.2010
(51) Int. Cl.: A61F 2/24, A61F 2/04, A61M 25/01, A61B 17/04

(54) **TISSUE PROTECTIVE DEVICE FOR CORONARY SINUS AND TRICUSPID VALVE FOR MITRAL VALVE CERCLAGE**
GEWEBESCHUTZVORRICHTUNG FÜR EINE KORONARSINUS- UND TRIKUSPIDAL-KLAPPE FÜR MITRALKLAPPEN-CERCLAGE
DISPOSITIF DE PROTECTION TISSULAIRE POUR SINUS CORONAIRE ET VALVULE TRICUSPIDE POUR LE CERCLAGE DE VALVULE MITRALE

(43) Date of publication of application: 07.08.2013
(73) Proprietor: Tau-PNU Medical Co., Ltd., Busan 46241 (KR)
(72) Inventor: KIM, June-Hong, Busan 48516 (KR)
(74) Representative: dompatent von Kreisler Selting Werner - Partnerschaft von Patent- und Rechtsanwälten mbB
(86) International application number: PCT/KR2010/006632
(87) International publication number: WO 2012/043898

(56) References cited:
- WO-A1-2006/105008
- WO-A2-02/100240
- KR-A- 20110 023 094
- US-A1- 2002 013 571
- US-A1- 2005 216 039
- US-A1- 2008 228 201

## Description

### [Technical Field]

The present invention relates, in general, to mitral valve cerclage annuloplasty apparatuses which are used during mitral valve cerclage annuloplasty performed on patients with mitral regurgitation, and which function to safely and effectively transfer and maintain a final appropriate tension required during mitral valve cerclage annuloplasty and, more particularly, to a tissue protective device that is used to protect the tissues of coronary sinus and tricuspid valve and adjacent tissues during mitral valve cerclage annuloplasty, configured to be used with a knot delivery device that delivers a knot of a cerclage suture to a top end of the tissue protective device so as to transfer and maintain the appropriate tension of the cerclage suture, and to a mitral valve cerclage annuloplasty apparatus that comprises the two devices.

### [Background Art]

Heart is the center of human circulatory system that pumps blood through our body. It is a muscle that pumps the blood only in one direction. In order for the heart to effectively maintain this unidirectional flow of blood, it must have properly functional valves that prevent back flow through its system, or regurgitation. Heart is divided into four chambers: right and left atrium, and right and left ventricles. The four chambers are connected to aorta, inferior and superior vena cava, pulmonary artery, and pulmonary veins.

Mitral valve (MV) separates left atrium from left ventricle while tricuspid valve (TV) separates right atrium from right ventricle. Aortic valve (AV) is located between left ventricle and aorta while pulmonary valve (PV) is located between right ventricle and pulmonary artery.

Generally, valves should open and close completely with every heart beat or contraction. Incomplete opening or closing of the valves causes improper flow of blood. Valvular diseases are divided into two categories, regurgitation and stenosis. Regurgitation is a failure of a valve to close completely and stenosis is a failure of a valve to open completely.

Mitral valve regurgitation (MVR) is a common cardiac valve disorder that is caused by incomplete closure of mitral valve (MV). MV is located between left atrium and left ventricle. Over time, MVR places burden on the heart and worsens its ability to pump blood properly. Such stress on the heart will ultimately lead to heart failure.

Traditional treatment of worsening MVR requires an open heart surgery with sternotomy or thoracotomy with cardiac arrest and cardio-pulmonary bypass. Once the chest is open and access to heart is gained, MV is either repaired or replaced using an artificial valve. Although very effective, this open-heart procedure is a high risk surgery accompanied by substantial morbidity and prolonged convalescence. Mortality due to surgery itself can be as high as 5%. As a result, the procedure often is not offered to patients who are insufficiently symptomatic to justify the surgical risk and morbidity, or to patients with substantial co-morbidity. It is reserved only for those with severe symptomatic MVR. This high morbidity rate of open heart surgery has motivated further research to develop a safer and less risky alternative to repair MVR. Much of the research involves use of cardiac catheterization.
WO 2006/105008 A1 discloses a device for treating a mitral heart valve that installs a bridge implant system comprising a bridging element sized and configured to span a left atrium between a great cardiac vein and an interatrial septum, a posterior bridge stop coupled to the bridging element and that abuts venous tissue within the great cardiac vein, an anterior bridge stop coupled to the bridging element and that abuts interatrial septum tissue in the right atrium, and a bridging element adjustment mechanism to shorten and/or lengthen the bridging element. At least one of the posterior bridge stop and the anterior bridge stop may include the bridging element adjustment mechanism.
Recently, the inventor of the present invention presented a thesis regarding "mitral valve cerclage coronary sinus annuloplasty (MCA)" showing outstanding results of MVR treatment through applying circular pressure around the mitral annulus. This thesis has been filed through PCT as an international patent application (PCT application number PCT/US2007/023836) and has been published (International publication number WO2008/060553).

The aforementioned thesis and published patent application disclosed the mitral cerclage coronary annuloplasty (MCA) procedure. Briefly explained, a catheter is placed in coronary sinus after accessing the right atrium through the jugular vein, and then a cerclage suture is passed through the proximal septal vein. This cerclage suture can easily pass through right ventricular outflow tract (RVOT), and this inventor defines this technique as "simple mitral cerclage annuloplasty." Then, the cerclage suture can be easily pulled into the right atrium thus placing the cerclage suture circumferentially around the mitral annulus. Once positioned, tension is applied to the cerclage suture, thereby tightening the mitral valve. This brings together the two leaves of MV so that they are approximated and reduce the size of its incomplete closure. This procedure can theoretically obtain very similar results to those that conventional surgeries can obtain by directly tightening the mitral annulus, and shows an immediate reduction of MVR.

However, there are several technical problems to be solved.

First, a tension locking device is required in order to be able to apply proper tension to the cerclage suture and to maintain the tension. According to the aforementioned research result, approximately 400-1200g of tension is needed to obtain a good treatment result. In addition, individualized tension must be applied constantly using MRI, X-ray inspection image, cardiac echography, etc. until the point where the mitral regurgitation is reduced, then, the suture needs to be fixed at that point so that the same tension is maintained. Further, this tension must be sustained regardless of constant resistance resulting from each beat of each heart contraction.

Second, since this tension is maintained with a very fine cerclage suture (i.e., 0.014" nylon cerclage was used in the research, but the thickness may be changed), it can cause damage to the neighboring cardiac tissues at points where the suture comes into contact with the tissues and exerts pressure. Especially, since the cerclage wraps around tricuspid valve (TV), it could affect the function of TV and damage the valve itself and its appendages. This invention is intended to provide viable solutions to overcome these problems of MCA.

### [Disclosure]

### [Technical Problem]

According to the invention is to overcome the shortcomings of conventional MCA. The scoop of the invention is defined in independent claim 1. Preferred embodiments of the invention are defined in the dependent claims.

The other objectives are to provide a protective device for tissues, which can prevent damage from occurring due to direct contact between tissue and cerclage suture. For example, it provides a knot delivery device (KDD) which allows an easier way of controlling, adjusting, and fixing the tension of the cerclage suture so that it can be customized for each individual patient. It further provides a tension locking function in which the fixed cerclage suture is maintained without becoming loose.

### [Technical Solution]

In order to accomplish the above objectives, in an aspect, the present invention provides a mitral valve cerclage annuloplasty apparatus configured to protect tissues of both coronary sinus and tricuspid valve and configured to deliver a knot of a cerclage suture so as to maintain a tension of the cerclage suture, the apparatus comprising: (A) a coronary sinus and tricuspid valve tissue protective device (CSTVPD) 20 comprising a coronary sinus tube (CS tube) 22, which is a hollow cylindrical tube for protecting tissues of the coronary sinus, a tricuspid valve tube (TV tube) 24, which is a hollow cylindrical tube for protecting tissues of the tricuspid valve and of interventricular septum, and a stem 26, in which the CS tube and the TV tube extend to be fixed and combined to each other; and (B) a knot delivery device (KDD) 30 that is a hollow catheter provided with an opening/closing part 32 in one end thereof and provided with a hole 34 formed longitudinally at a side of the opening/closing part.

In a preferred embodiment, the TV tube 24 has a stopper 24a that is positioned about distal part of the TV tube to prevent further advancement of the TV tube into a cardiac muscle.

In a preferred embodiment, the TV tube has a tapering shape towards an end thereof such that the TV tube can cover the cerclage suture inside the interventricular septum.

In a preferred embodiment, the CS tube 22 is made of a soft and flexible material, and the TV tube 24 and the stem 26 are made of materials that are more rigid compared to the material of the CS tube.

For example, the opening/closing part of the knot delivery device (KDD) is opened by being cut using a cutter.

In another example, the knot delivery device (KDD) 30 comprises a first tube 36 that functions as an external tube and a second tube 38 that is rotatably placed in the first tube, wherein the opening/closing part 32 is opened or closed by a rotation of the second tube 38. More preferably, the first tube 36 is provided with an opening 37 in an end thereof, in which a length of the opening 37 is equal to a sum total of a length L1 of the opening/closing part and a length L2 of the hole, and the second tube 38 is provided with an opening 39 having a length greater than the length of the opening 37 of the first tube, in which the opening 39 comprises an entering part 39a that has a length equal to the length L1 of the opening/closing part and has a width d2 greater than a width d1 of the opening/closing part, and an internal part 39b that has a length greater than the length L2 of the hole and has a width d3 greater than a sum total of the width d2 of the entering part and the width d1 of the hole.

### [Advantageous Effects]

As described above, the mitral valve cerclage annuloplasty apparatus of the present invention comprises a tissue protective device

The tissue protective device can prevent the cerclage suture from damaging the tissues of coronary sinus and tricuspid valve, and a knot delivery device , which is not claimed, can deliver and maintain the knot of the cerclage suture, and can control the tension of the cerclage suture, and can continuously maintain the tension of the cerclage suture after controlling the tension.

### [Description of Drawings]

Fig. 1 is a schematic view of a tissue protective device for protecting the tissues of coronary sinus and tricuspid valve according to a preferred embodiment of the present invention;
Fig. 2 is a front sectional view of a human heart, in which the tissue protective device according to the preferred embodiment of the present invention is placed;
Fig. 3 is a side sectional view of the human heart, in which the tissue protective device according to the preferred embodiment of the present invention is placed;
Fig. 4 is a schematic view of a knot delivery device according to the preferred embodiment of the present invention;
Fig. 5 a mitral valve cerclage annuloplasty apparatus according to the preferred embodiment of the present invention; and
Fig. 6 is a schematic view of a knot delivery device .

*** Description of reference characters of important parts ***

| | | | |
|---|---|---|---|
| 10: | cerclage suture | 12: | knot |
| 20: | tissue protective device | | |
| 22: | coronary sinus tube (CS tube) | | |
| 24: | tricuspid valve tube (TV tube) | | |
| 24a: | RVOT exit stopper | | |
| 26: | stem | | |
| 27: | hinge portion | | |
| 30: | knot delivery device | 32: | opening/closing part |
| 34: | hole | | |
| 36: | first tube | 37: | opening |
| 38: | second tube | 39: | opening |
| 39a: | entering part | 39b: | internal part |
| 40: | coronary protective device | | |

### [Best Mode]

Hereinbelow, a tissue protective device for protecting tissues of coronary sinus and tricuspid valve and a knot delivery device, which are used in mitral valve cerclage annuloplasty, and a mitral valve cerclage annuloplasty apparatus comprising the tissue protective device and the knot delivery device will be described in detail with reference to the accompanying drawings.

First, the coronary sinus and tricuspid valve tissue protective device (CSTVPD) 20 used in mitral valve cerclage annuloplasty will be described.

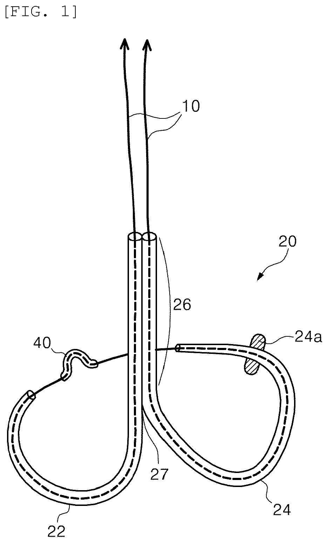

Fig. 1 is a schematic view of CSTVPD according to a preferred embodiment of the present invention, in which the CSTVPD 20 that is inserted into a cerclage suture 10 is illustrated.

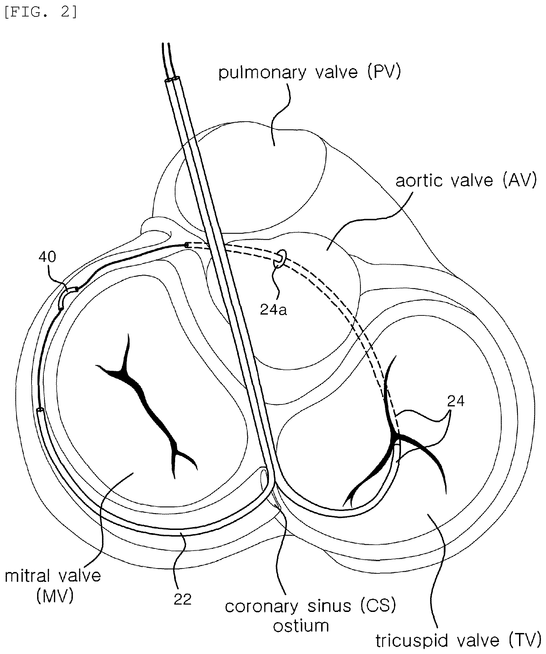

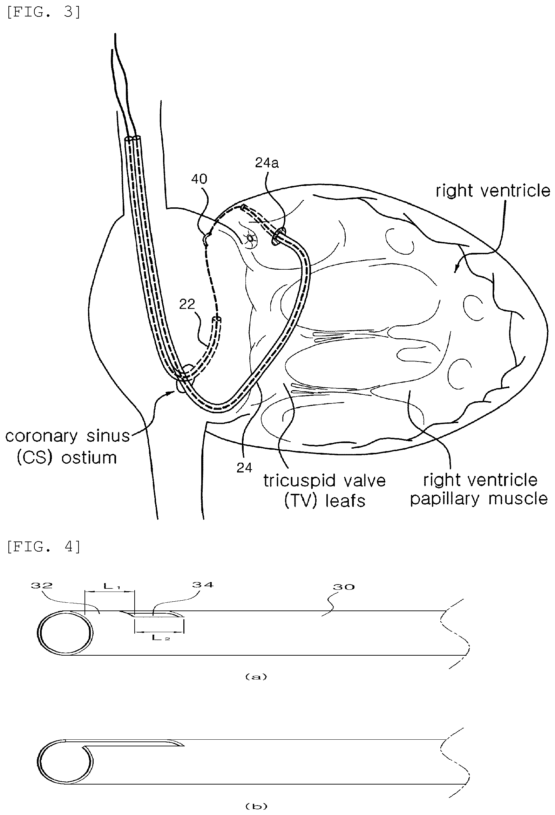

Fig. 2 is a front sectional view of a human heart, in which the CSTVPD according to the preferred embodiment of the present invention is placed, and Fig. 3 is a side sectional view of the human heart.

The cerclage suture 10 is a thin biocompatible suture that is used in mitral valve cerclage annuloplasty (MCA), in which a piece of suture passes through coronary sinus (CS), tricuspid valve (TV) and interventricular septum in a circle form, and comes out of the body, and so the suture was named as cerclage suture. When the cerclage suture comes out of the body, two strands of suture that are the opposite ends of the suture come out of the body.

According to FIG. 1 or 3, CSTVPD 20 of the present invention comprises coronary sinus tube 22 (CS tube), which is a hollow cylindrical tube for protecting coronary sinus tissues, tricuspid valve tube 24 (TV tube), which is a hollow cylindrical tube for protecting the tissues of tricuspid valve and interventricular septum (cardiac muscle), and stem 26. Here, CS tube 22 encircles CS, and TV tube 24 encircles TV and the extensions of CS tube 22 and TV tube 24 meet and run together to form stem 26.

Generally, conventional MCA techniques cause tissue damage (erosion) to CS, TV, and interventricular septum (IVS) from direct contact between cerclage suture and tissue. These critical structures can be protected from damage using the CSTVPD 20 of the present invention around the cerclage suture 10.

A part of CS is protected by coronary protective device 40 (CPD) introduced in the previous MCA thesis. Thus, only the remainder of CS will need to be protected.

CSTVPD 20 of the present invention has two separated tubes which are separated at lower ends and joined together at upper ends. The two tubes of CSTVPD are hollow cylindrical tubes having hollow spaces. The thickness of the tubes is similar to 4Fr diagnostic catheters and the tubes are made of flexible material such as rubber, plastic etc. The inventor defines TPD 20 as "CSTV protective device (CSTVPD)" or "CSTV" since it protects both CS and TV.

During mitral valve cerclage annuloplasty (MCA), two strands of cerclage suture 10 that come out of the body are inserted into CS tube 22 and into TV tube 24, respectively, and the two tubes are inserted into the body using a catheter, etc.

Then, CS tube 22 of CSTVPD 20 is positioned to wrap around CS, and TV tube 24 is positioned to wrap around TV. Since the cerclage suture 10 is inside the tubes and not in direct contact with the surrounding tissue, cardiac tissues around CS, TV and IVS, but the outer surface of the CSTVPD 20 comes into contact with the tissues, and so the tissues are protected from damage (erosion) from the cerclage suture 10.

Hereinbelow, CSTVPD 20 will be described in more detail as follows.

CS tube 22 is a part of a coronary sinus (CS) protection system. CS tube 22 starts at the orifice of CS to the beginning of the coronary protective device (CPD) 40. Anatomically, this length varies from patient to patient. Thus, before the procedure, appropriate length can be determined using estimation from CT (cardiac CT) or other imaging methods. CS tube 22 should be made of soft and flexible catheter-like material so that its affect on compression of CS is minimized.

TV tube 24 has a tapering shape towards end such that it can cover the cerclage suture inside interventricular septum (cardiac muscle). After starting from the stopper, TV tube 24 passes through the muscle of IVS (cardiac muscle). Therefore, in order to allow easy passing through the IVS muscle, TV tube 24 shall taper from stopper 24a to the end.

TV tube 24 has a ring-shaped stopper (RVOT exit stopper 24a) that protrudes to form a ring shape and is positioned about distal part of TV tube 24 to prevent further advancement of TV tube 24 into the cardiac muscle (interventricular septum). Further, the length of TV tube 24 shall vary from patient to patient. The length shall be determined based on estimation based on prior imaging studies of each individual patient. Here, it is required to previously select the lengths of the RVOT exit stopper and the hinge such that the length of TV tube 24 shall be about twice the length of the distance from RVOT to the orifice of CS measured using images of the patient. That is, the length of TV tube 24 from hinge 27 to the stopper 24a shall be longer than the distance from stopper 24a to the tapered end of TV tube 24. To this end, the location of stopper 24a may vary inside TV tube 24 as needed. Alternatively, several kinds of TV tubes having different lengths from the hinge to the stopper 24a may be provided. Hinge 27, where CS tube 22 and TV tube 24 meets, shall be placed at or near the orifice of CS. TV tube 24 will be fixed to the heart at hinge 27 and at stopper (RVOT exit stopper) 24a. Then, hinge 27 to stopper 24a portion of TV tube 24 can be suspended freely in a reverse "C" shape without being directly attached to the TV tissue. Such a technique can reduce TV tissue damage (erosion) resulting from direct contact with cerclage suture 10 and it also reduces the restriction of cerclage on movement of TV leaflets.

TV tube 24 shall be rigid enough to resist being bent (kinked) as tension is applied to the cerclage suture. On the other hand, it shall be flexible enough to bend in a reverse "C" shape.

Stem 26 is a part at which the CS tube and the TV tube respectively extend and join together, and ends thereof are located in opposite veins (see Fig. 3). Stem 26 plays roles in which stem 26 stabilizes CS tube 22 and TV tube 24 so that their positions are well maintained. Further, since hinge 27 at which CS tube 22 and TV tube 24 join together rests on the orifice of the CS, it prevents further advancement of CSTVPD 20 into the CS. Stem 26 (where CS tube 22 and TV tube 24 join together) should be made of a semi-rigid catheter-like material.

Fig. 4 is a schematic view of a knot delivery device (KDD) .

KDD 30 is designed to transfer cerclage suture 10 looped and ready to be knotted to the upper portion of CSTVPD 20 while maintaining constant tension on the cerclage suture. Once position and appropriate tension is verified, KDD 30 will release the loop and knot of the suture in place thus ensuring the knot is positioned in the right place with an appropriate amount of tension.

One of the most crucial components of MCA procedure is having a device that can deliver and maintain enough necessary tension to apply compression on mitral annulus. That device should (1) be easy to operate, (2) allow for easy manipulation of cerclage suture tension depending on individual patient variance, (3) be easy to readjust and fix tension, and (4) once cerclage suture is fixed, not become loose, and maintain constant tension well after the procedure has been completed.

FIG. 4 shows an example of KDD where (a) is in closed position and (b) is in open position.

KDD 30 is a catheter where opening/closing part 32 can be in a closed position (a) to create hole 34 or be in an opened position (b) to connect part 32 to hole 34 into a continuous slit like opening. KDD 30 should be made of a sturdy material often used in diagnostic catheters. It can be made of a rubber- or plastic-like material strong enough to be pushed inside the body from the outside.

As in FIG. 4(a) where the opening/closing part is in a closed position, the cerclage suture is made into a loop ready to be knotted at the end of the KDD. Then, the KDD with the loosely knotted cerclage suture is advanced inside the body and positioned appropriately. Then, tension is applied and adjusted. When position and tension are verified, the opening/closing part is opened as in FIG. 4(b), fixing the knot in place while maintaining the same amount of tension on the cerclage suture.

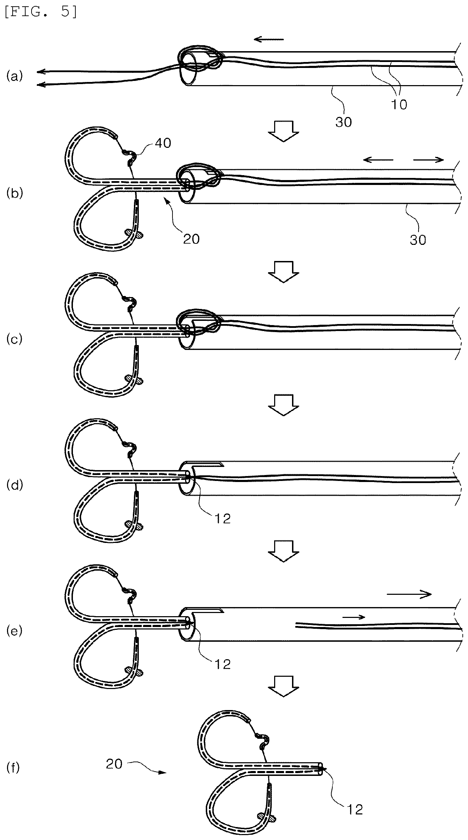

Hereinbelow, KDD 30 will be described in more detail with reference to Fig. 5.

Fig. 5 is a view showing the use of the mitral valve cerclage annuloplasty apparatus according to the preferred embodiment of the present invention.

As shown in Fig. 5, first, the two stands of cerclage suture 10 that come out of the body are inserted through the hole 34 at the end of KDD 30. Second, the two strands of suture are looped and made ready to form a knot. Third, the two strands of suture are passed through the body of KDD 30. (Fig. 5a)

Then, KDD30 is advanced into heart and the end of KDD 30 is positioned at the top end of CSTVPD 20. Hence, KDD 30 functions to deliver cerclage suture knot to CSTVPD 20 through hole 34 while keeping the knot from tightening until it is ready to be fixed.

Thereafter, appropriate amount of tension is applied to cerclage suture 10 and adjusted until mitral regurgitation ceases. In other words, tension on the cerclage suture 10 is adjusted by pulling or releasing the respective ends of the two strands of suture until the mitral regurgitation stops. (Fig. 5b)

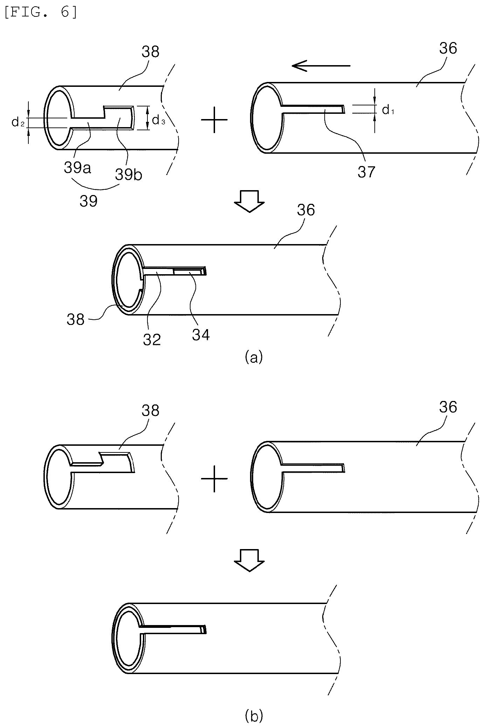

Once a desired amount of tension is achieved, opening/closing part 32 of KDD 30 is opened. Opening of KDD 30 may be cut with a cutter or various methods (not claimed) may be utilized as will be explained later herein with reference to Fig. 6.

When opening/closing part 32 opens, the loop tightens into a knot while maintaining the desired amount of tension on the cerclage suture (Fig. 5d) so that the end of cerclage suture 10 is fixed by knot 12. Once knot 12 is placed and tightened at the end of the stem, the stem can now hold the two strands of cerclage suture aligned and in place. This also enables the knot to efficiently resist becoming loose even under a significant amount of tension.

At this stage, the cerclage suture 10 is cut at a certain distance from the knot with a cutter. And then, the remainder of the cerclage suture 10 and KDD 30 is retracted from the body. (Fig. 5e)

Thus, cerclage suture 10 wraps CS and TV by CSTVPD 20 while maintaining constant tension, thereby finishing the mitral cerclage coronary annuloplasty (MCA) procedure.

Fig. 6 is a schematic view of KDD according to another example of the present invention. KDD of Fig. 6 uses a different method (not claimed) than cutting the open-close part.

As shown in Fig. 6, KDD 30 of this example comprises first tube 36 that functions as an external tube and second tube 38 that is rotatably placed in the first tube and functions as an internal tube, and so opening/closing part 32 can be opened or closed by a rotation of first tube 36 or second tube 38. Here, the first tube and the second tube are made of basic catheter-type of material.

FIG. 6a shows opening/closing part 32 in closed position. Opening/closing part 32 can be opened or closed as first tube 36 or second tube 38 is rotated. FIG. 6b shows the opening/closing part in an opened state.

As shown in the drawings, in an end of first tube 36, opening 37 is formed, in which the length of opening 37 is equal to the sum total of length L1 of the opening/closing part and length L2 of the hole. Here, width d1 of opening 37 is equal to the width of the opening/closing part and to the width of the hole.

Further, second tube 38 is provided with opening 39. Here, the length of opening 39 is greater than the length of opening 37 of the first tube. Opening 39 comprises entering part 39a and internal part 39b. Entering part 39a has a length equal to length L1 of the opening/closing part and has a width d2 greater than width d1 of the opening/closing part. Internal part 39b has a length greater than length L2 of the hole and has width d3 greater than the sum total of width d2 of the entering part and width d1 of the hole.

Accordingly, when either first tube or second tube rotated in a state in which the opening/closing part is closed, the opening/closing part is brought into an open state, and so KDD 20 can be used as a knot delivery system.

### [Industrial Applicability]

As described above, the present invention provides a mitral valve cerclage annuloplasty apparatus which can be used during mitral valve cerclage annuloplasty (MCA) performed on mitral valve cerclage patients with mitral regurgitation and which can function to safely and effectively transfer and maintain a final appropriate tension required during mitral valve cerclage annuloplasty. Especially, the present invention provides a tissue protective device (CSTVPD) that is used to protect the tissues of coronary sinus (CS) and tricuspid valve (TV) and peripheral tissues during mitral valve cerclage annuloplasty.

## Claims

1. A coronary sinus and tricuspid valve tissue protective device (20) for protecting tissues of coronary sinus and tricuspid valve during mitral valve cerclage annuloplasty, the coronary sinus and tricuspid valve tissue protective device (20) comprising:
a coronary sinus tube (22) that is a hollow cylindrical tube for protecting tissues of the coronary sinus; and
a tricuspid valve tube (24) that is a hollow cylindrical tube for protecting tissues of the tricuspid valve and of interventricular septum;
**characterized in that**
the coronary sinus and tricuspid valve tissue protective device (20) further comprises a stem (26) in which the coronary sinus tube (22) and the tricuspid valve tube (24) extend to be fixed and combined to each other.

2. The coronary sinus and tricuspid valve tissue protective device (CSTVPD) as set forth in claim 1, wherein the tricuspid valve tube (24) has a stopper (24a) that is positioned about distal part of the tricuspid valve tube (24) to prevent further advancement of the tricuspid valve tube (24) into a cardiac muscle.

3. The coronary sinus and tricuspid valve tissue protective device (20) as set forth in claim 2, wherein the stopper (24a) protrudes so as to form a ring-shaped stopper.

4. The coronary sinus and tricuspid valve tissue protective device (20) as set forth in claim 1, wherein the tricuspid valve tube (24) has a tapering shape towards an end thereof such that the tricuspid valve tube (24) can cover the cerclage suture inside the interventricular septum.

5. The coronary sinus and tricuspid valve tissue protective device (20) as set forth in claim 1, wherein the coronary sinus tube (22) is made of a soft and flexible material, and the tricuspid valve tube (24) and the stem (26) are made of materials that are more rigid compared to the material of the coronary sinus tube (22).

6. A tissue protective device used in mitral valve cerclage annuloplasty as set forth in claim 1, the device comprising:
the coronary sinus tube (22) which is a first protective tube having a proximal end, a distal end, and an opening extending therebetween to receive a portion of a cerclage suture (10) therein; and
the tricuspid valve tube (24) which is a second protective tube having a proximal end, a distal end, and an opening extending therebetween to receive another portion of the cerclage suture (10) therein, wherein the proximal end of the first protective tube and the proximal end of the second protective tube are attached side-by-side longitudinally along at least a portion of the respective proximal ends embodying the stem (26), and the openings of the distal ends of the first protective tube and the second protective tube are generally directed toward one another.

7. The device according to claim 6, wherein the tissue protective device further comprises a ring-shaped stopper being positioned on the distal portion of the second protective tube to prevent further advancement of the tube into a heart muscle.

8. The device according to claim 6, wherein the distal portion of the second protective tube tapers to an end.

9. The device according to claim 6, wherein the first protective tube and the second protective tube are made of a flexible material.

10. The device according to claim 6, wherein the first protective tube and the second protective tube have a thickness similar to a 4 Fr diagnostic catheter.

11. The device according to claim 6, wherein each of the distal portions of the first protective tube and the second protective tube has an arched configuration so that the distal portion can be positioned to encircle the coronary sinus.

## Patentansprüche

1. Koronarsinus- und Trikuspidalklappengewebe-Schutzvorrichtung (20) zum Schützen der Gewebe der Koronarsinus- und Trikuspidalklappe während einer Mitralklappen-Cerclage-Annuloplastie, wobei die Koronarsinus- und Trikuspidalklappengewebe-Schutzvorrichtung (20) umfasst:
ein Koronarsinusrohr (22), das ein hohlzylinderförmiges Rohr zum Schützen der Gewebe des Koronarsinus ist; und
ein Trikuspidalklappenrohr (24), das ein hohlzylinderförmiges Rohr zum Schützen der Gewebe der Trikuspidalklappe und des Kammerseptums ist;
**dadurch gekennzeichnet, dass**
die Koronarsinus- und Trikuspidalklappengewebe-Schutzvorrichtung (20) weiterhin einen Schaft (26) umfasst, in den hinein sich das Koronarsinusrohr (22) und das Trikuspidalklappenrohr (24) erstrecken, um befestigt und miteinander kombiniert zu werden.

2. Koronarsinus- und Trikuspidalklappengewebe-Schutzvorrichtung (CSTVPD) gemäß Anspruch 1, wobei das Trikuspidalklappenrohr (24) einen Stopper (24a) aufweist, der sich um den distalen Teil des Trikuspidalklappenrohrs (24) herum befindet, um ein weiteres Vordringen des Trikuspidalklappenrohrs (24) in einen Herzmuskel zu verhindern.

3. Koronarsinus- und Trikuspidalklappengewebe-Schutzvorrichtung (20) gemäß Anspruch 2, wobei der Stopper (24a) hervorsteht, so dass er einen ringförmigen Stopper bildet.

4. Koronarsinus- und Trikuspidalklappengewebe-Schutzvorrichtung (20) gemäß Anspruch 1, wobei das Trikuspidalklappenrohr (24) eine sich zu einem seiner Enden hin verjüngende Form aufweist, so dass das Trikuspidalklappenrohr (24) die Cerclage-Naht innerhalb des Kammerseptums bedecken kann.

5. Koronarsinus- und Trikuspidalklappengewebe-Schutzvorrichtung (20) gemäß Anspruch 1, wobei das Koronarsinusrohr (22) aus einem weichen und flexiblen Material besteht und das Trikuspidalklappenrohr (24) und der Schaft (26) aus Materialien bestehen, die steifer sind als das Material des Koronarsinusrohrs (22).

6. Gewebeschutzvorrichtung, die bei der Mitralklappen-Cerclage-Annuloplastie verwendet wird, gemäß Anspruch 1, wobei die Vorrichtung umfasst:
das Koronarsinusrohr (22), bei dem es sich um ein erstes Schutzrohr mit einem proximalen Ende, einem distalen Ende und einer sich dazwischen erstreckenden Öffnung handelt, um einen Teil einer Cerclage-Naht (10) darin aufzunehmen; und
das Trikuspidalklappenrohr (24) bei dem es sich um ein zweites Schutzrohr mit einem proximalen Ende, einem distalen Ende und einer sich dazwischen erstreckenden Öffnung handelt, um einen anderen Teil der Cerclage-Naht (10) darin aufzunehmen, wobei das proximale Ende des ersten Schutzrohrs und das proximale Ende des zweiten Schutzrohrs längs nebeneinander entlang wenigstens einem Teil der jeweiligen proximalen Enden, die den Schaft (26) verkörpern, befestigt sind und die Öffnungen der distalen Enden des ersten Schutzrohrs und des zweiten Schutzrohrs im Wesentlichen zueinander hin gerichtet sind.

7. Vorrichtung gemäß Anspruch 6, wobei die Gewebeschutzvorrichtung weiterhin einen ringförmigen Stopper umfasst, der sich an dem distalen Teil des zweiten Schutzrohrs befindet, um ein weiteres Vordringen des Rohrs in einen Herzmuskel zu verhindern.

8. Vorrichtung gemäß Anspruch 6, wobei sich der distale Teil des zweiten Schutzrohrs zu einem Ende hin verjüngt.

9. Vorrichtung gemäß Anspruch 6, wobei das erste Schutzrohr und das zweite Schutzrohr aus einem flexiblen Material bestehen.

10. Vorrichtung gemäß Anspruch 6, wobei das erste Schutzrohr und das zweite Schutzrohr eine ähnliche Dicke aufweisen wie ein 4-Fr-Diagnosekatheter.

11. Vorrichtung gemäß Anspruch 6, wobei die distalen Teile des ersten Schutzrohrs und des zweiten Schutzrohrs jeweils eine gewölbte Konfiguration aufweisen, so dass der distale Teil so positioniert sein kann, dass er den Koronarsinus umgibt.

## Revendications

1. Dispositif protecteur de tissu de sinus coronaire et de valve tricuspide (20) destiné à protéger des tissus de sinus coronaire et de valve tricuspide pendant une annuloplastie à cerclage de valve mitrale, le dispositif protecteur de tissu de sinus coronaire et de valve tricuspide (20) comprenant :
un tube de sinus coronaire (22) qui est un tube cylindrique creux destiné à protéger des tissus du sinus coronaire ; et
un tube de valve tricuspide (24) qui est un tube cylindrique creux destiné à protéger des tissus de la valve tricuspide et du septum interventriculaire ;
**caractérisé en ce que**
le dispositif protecteur de tissu de sinus coronaire et de valve tricuspide (20) comprend en outre une tige (26) dans laquelle le tube de sinus coronaire (22) et le tube de valve tricuspide (24) s'étendent pour être fixés et combinés l'un à l'autre.

2. Dispositif protecteur de tissu de sinus coronaire et de valve tricuspide (CSTVPD) selon la revendication 1, dans lequel le tube de valve tricuspide (24) comporte un obturateur (24a) qui est positionné autour d'une partie distale du tube de valve tricuspide (24) pour empêcher le tube de valve tricuspide (24) de s'avancer plus avant dans un muscle cardiaque.

3. Dispositif protecteur de tissu de sinus coronaire et de valve tricuspide (20) selon la revendication 2, dans lequel l'obturateur (24a) dépasse de manière à former un obturateur en forme de bague.

4. Dispositif protecteur de tissu de sinus coronaire et de valve tricuspide (20) selon la revendication 1, dans lequel le tube de valve tricuspide (24) a une forme effilée vers une de ses extrémités de telle sorte que le tube de valve tricuspide (24) peut couvrir la suture de cerclage à l'intérieur du septum interventriculaire.

5. Dispositif protecteur de tissu de sinus coronaire et de valve tricuspide (20) selon la revendication 1, dans lequel le tube de sinus coronaire (22) comprend un matériau souple et flexible, et le tube de valve tricuspide (24) et la tige (26) comprennent des matériaux qui sont plus rigides en comparaison au matériau du tube de sinus coronaire (22).

6. Dispositif protecteur de tissu utilisé dans une annuloplastie à cerclage de valve mitrale selon la revendication 1, le dispositif comprenant :
le tube de sinus coronaire (22) qui est un premier tube protecteur ayant une extrémité proximale, une extrémité distale, et une ouverture s'étendant entre celles-ci pour y recevoir une portion d'une suture de cerclage (10) ; et
le tube de valve tricuspide (24) qui est un second tube protecteur ayant une extrémité proximale, une extrémité distale, et une ouverture s'étendant entre celles-ci pour y recevoir une autre portion de la suture de cerclage (10), dans lequel l'extrémité proximale du premier tube protecteur et l'extrémité proximale du second tube protecteur sont attachées côte à côte longitudinalement le long d'au moins une portion des extrémités proximales respectives incarnant la tige (26), et les ouvertures des extrémités distales du premier tube protecteur et du second tube protecteur sont généralement dirigées l'une vers l'autre.

7. Dispositif selon la revendication 6, dans lequel le dispositif protecteur de tissu comprend en outre un obturateur en forme de bague qui est positionné sur la portion distale du second tube protecteur pour empêcher le tube d'avancer plus avant dans un muscle du cœur.

8. Dispositif selon la revendication 6, dans lequel la portion distale du second tube protecteur s'effile vers une extrémité.

9. Dispositif selon la revendication 6, dans lequel le premier tube protecteur et le second tube protecteur comprennent un matériau flexible.

10. Dispositif selon la revendication 6, dans lequel le premier tube protecteur et le second tube protecteur ont une épaisseur similaire à un cathéter de diagnostic 4 Fr.

11. Dispositif selon la revendication 6, dans lequel chacune des portions distales du premier tube protecteur et du second tube protecteur a une configuration arquée de telle sorte que la portion distale peut être positionnée pour encercler le sinus coronaire.
